# EUROPEAN PATENT SPECIFICATION

(11) **EP 0 321 273 B1**
(45) Date of publication and mention of the grant of the patent: **20.04.1994**
(21) Application number: 88311926.5
(22) Date of filing: 16.12.1988
(51) Int. Cl.: C07C 327/38, A61K 31/16, A61K 7/06

(54) **Thioformamide derivatives**
Thioformamid-Derivate
Dérivés de thioformamide

(30) Priority: 18.12.1987 GB 8729522
(43) Date of publication of application: 21.06.1989
(73) Proprietor: RHONE-POULENC RORER LIMITED, West Malling, Kent ME19 4TA (GB)
(72) Inventor: Cook, David Charles, Dagenham Essex, RM10 7XS (GB); Hart, Terance William, Dagenham Essex, RM10 7XS (GB); Mclay, Iain McFarlane, Dagenham Essex, RM10 7XS (GB); Palfreyman, Malcolm Norman, Dagenham Essex, RM10 7XS (GB); Walsh, Roger John Aitchison, Dagenham Essex, RM10 7XS (GB)
(74) Representative: Bentham, Stephen

(56) References cited:
- No relevant documents have been disclosed.

## Description

This invention relates to new therapeutically useful thioformamide derivatives, to processes for their preparation and to pharmaceutical compositions containing them.

The new thioformamide derivatives of the present invention are those compounds of the general formula (I) hereinafter depicted, wherein R represents a straight- or branched-chain alkyl radical containing from 1 to 4 carbon atoms, Ar represents
a phenyl group unsubstituted or substituted in the 3 and/or 5 position with an electron-withdrawing group for example a cyano, nitro, trifluoromethyl, carbamoyl, carboxy, C₂₋₅-alkanoyl, C₂₋₅-alkoxycarbonyl or C₁₋₄-alkylsulphonyl group or a fluorine, chlorine or bromine atom, and optionally further substituted with halogen atom(s), C ₁₋₄-alkyl or C₆₋₁₂-aryl (e.g. phenyl) group(s), or
the group Ar may be substituted with halogen atom(s), C₁₋₄-alkyl or C₆₋₁₂-aryl (e.g. phenyl) group(s) or with substituents which together form a fused ring, for example a 2-naphthyl group, Y represents a valency bond or an ethylene or preferably methylene radical, and X represents a carbonyl or hydroxymethylene group or a group of the formula: >C=NOR¹, >C=NN(R¹)₂ or >C=NN(R¹)CON(R¹)₂ in which the symbols R¹, which may be the same or different, each represents the hydrogen atom or a straight- or branched-chain alkyl radical containing from 1 to 4 carbon atoms which is unsubstituted or substituted by one or more substituents selected from C₂₋₄-alkenyl, carboxy, C₂₋₅-alkoxycarbonyl, hydroxy, C₁₋₄-alkoxy, carbamoyl (unsubstituted or substituted by one or two C₁₋₄-alkyl groups), amino, C₁₋₄-alkylamino and di-C₁₋₄-alkylamino groups (e.g. R¹ may represent a methyl, 2-hydroxy-3-isopropyl-aminopropyl or 2-hydroxy-3-t.butylamino-propyl radical), or represents a benzyl, phenethyl, 1-naphthylmethyl, 2-naphthylmethyl or pyrid-3-ylmethyl radical each of which may be substituted on the ring by one or more halogen atoms or hydroxy, C₁₋₄-alkyl, C₁₋₄-alkoxy (alkoxy being unsubstituted or substituted as defined for alkyl groups represented by R¹), cyano, nitro, trifluoromethyl, carboxy, C₁₋₄-alkylamino, C₂₋₅-alkanoylamino or C₂₋₅-alkoxycarbonyl groups or two R¹ substituents on the same nitrogen atom may together form a straight- or branched-chain alkylene radical containing from 4 to 6 carbon atoms in the chain which is unsubstituted or substituted as defined for alkyl radicals represented by R¹ (e.g. 1-methoxymethyltetramethylene) and pharmaceutically acceptable salts thereof.

Preferably X represents the carbonyl group or a group of formula >C=NOR¹ as hereinbefore defined.

The group Ar may represent, for example, the phenyl, 4-chlorophenyl, 3-trifluoromethylphenyl, 3-nitrophenyl, 3,4-dichlorophenyl or 2-naphthyl group.

The presence of a hydroxy group on the ring creates an asymmetry in the molecule which, in association with the adjacent asymmetric carbon atom, leads to 4 stereoisomers which, optionally, can be separated into 2 racemic pairs. The racemic pair and its enantiomers of the general formula (II) in which R, Ar and Y as hereinbefore defined, i.e. the compounds in which the hydroxy group is in the trans position relative to the group -CSNHR are preferred.

Furthermore, in certain cases the substituents R and R¹ contribute to stereoisomerism. All such forms are embraced by the present invention.

Particular compounds of the present invention are as follows:
A (±)-N-methyl-2-oxo-1-phenylcyclohexanecarbothioamide
B (±)-trans-N-methyl-2-hydroxy-1-phenylcyclohexanecarbothioamide
C (±)-anti-N-methyl-2-hydroxyimino-1-phenylcyclohexanecarbothioamide
D (±)-anti-N-methyl-2-methoxyimino-1-phenylcyclohexanecarbothioamide
E (±)-N-methyl-2-oxo-1-(4-chlorophenyl)cyclohexanecarbothioamide
F (±)-N-methyl-2-oxo-1-(3-trifluoromethylphenyl)cyclohexanecarbothioamide
G (±)-anti-N-methyl-2-benzyloxyimino-1-(3,4-dichlorophenyl)cyclohexanecarbothioamide
H (±)-anti-N-methyl-2-(4-fluorobenzyloxyimino)-1-phenylcyclohexanecarbothioamide,
I (±)-N-methyl-2-oxo-1-(2-naphthyl)cyclohexanecarbothioamide
J (±)-N-methyl-2-oxo-1-(3,4-dichlorophenyl)cyclohexanecarbothioamide
K (±)-N-methyl-2-oxo-1-(3-nitrophenyl)cyclohexanecarbothioamide
as well as their enantiomeric and diastereoisomeric and syn forms, where they exist.

The letters A to K are allocated to the compounds for easy reference later in the specification, e.g. in the Table and in the Examples.

The compounds have valuable pharmacological properties, in particular properties which are indicative of utility in the treatment and/or prophylaxis of disorders associated with:-
(1) vascular smooth muscle contraction including hypertension and other cardiovascular disorders such as congestive heart failure, and conditions associated with tissue ischaemia such as angina, peripheral vascular disease and cerebrovascular disease;
(2) respiratory smooth muscle contraction including reversible airways obstruction and asthma;
(3) contraction of smooth muscle of gastrointestinal tract, urinary bladder and uterus, including peptic ulcers, irritable bowel syndrome and diverticular disease; irritable bladder syndrome; and premature labour.

The compounds also have utility in the inhibition of head hair loss associated with male pattern baldness, by topical application.

For example, compounds of general formula (I) were submitted to:-

### Vaso-relaxant Activity Tests.

The test methods used were adapted from those described by Winslow et al [Eur.J.Pharmacol., 131, 219-228 (1986)] and Karaki [J.Pharmacol. Methods, 18, 1-21 (1987)] for differentiating vaso-relaxant activity.

### Test A : Activity against contractions induced by low K⁺ concentrations in the isolated rat aorta

Thoracic aorta was removed from rats and transverse strips, denuded of endothelium, were suspended in a bath containing Krebs solution. The tension was recorded and a contraction induced by addition of 20 mM K⁺ (potassium ion) to the bathing solution. The test compound was added to the bath as a solution in increasing cumulative concentration. The concentration in the bathing solution of the test compound which reduced the K⁺-induced contraction by 90% was determined and expressed in µM as the effective concentration (EC₉₀), given in Table I.

### Test B : Activity against contractions induced by high K⁺ concentrations in isolated rat aorta

The test method was as in Test A except that contractions were induced by addition of 60 mM K⁺ to the bathing solution. The cumulative addition of solutions of the test compound was conducted and the concentration in the bath reducing the K⁺-induced contraction by 90% was greater than 30µM for Compounds C, D, E, F, I, J and K, and much greater than 30µM for Compound A.

**Table I**

| Compound | Activity Test A EC₉₀ µM |
|---|---|
| A | 29 |
| C | >30 |
| D | 20 |
| E | 12 |
| F | 0.9 |
| H | 0.6 |
| I | 2.0 |
| J | 0.4 |
| K | 0.7 |

The compounds of general formula (I) can be prepared by the application or adaptation of known methods, for example as hereinafter identified. By the term "known methods" as used in this specification is meant methods heretofore used or described in the literature.

According to a feature of the present invention, the compounds of general formula (I) wherein X represents the carbonyl group or a group of formula >C=NOR¹ or >C=NN(R¹)₂ wherein R¹ is as hereinbefore defined may be prepared by the reaction of a compound of general formula (III) wherein X′ represents the carbonyl group or a group of formula >C=NOR¹ or >C=NN(R¹)₂ as hereinbefore defined and Ar and Y are as hereinbefore defined with an isothiocyanate of the general formula:

R-N=C=S (IV)

wherein R represents a straight- or branched-chain alkyl radical containing 1 to 4 carbon atoms.

The reaction is generally carried out in an anhydrous inert organic solvent such as tetrahydrofuran, dimethyl formamide or hexamethylphosphoramide, or a mixture of these solvents, at a temperature from -80°C to +50°C, in the presence of an organic base such as potassium tert.-butoxide or an organo-lithium derivative such as butyllithium, or of sodium hydride.

According to a feature of the present invention, the compounds of general formula (I) wherein X represents the hydroxymethylene group may be prepared by the reduction of a compound of general formula (I) wherein X represents the carbonyl group.

The reduction is generally carried out in an inert organic solvent such as methanol or dimethylsulphoxide, or a mixture of these solvents at a temperature from -20°C to +50°C, using an alkali metal borohydride, e.g. sodium borohydride.

According to a feature of the present invention, the compounds of general formula (I) wherein X represents a group of the formula: >C=NOR¹, >C=NN(R¹)₂ or >C=NN(R¹)CON(R¹)₂ as hereinbefore defined may be prepared by the reaction of a compound of general formula (I) wherein X represents the carbonyl group with a compound of general formula:

NH₂OR¹ (Va)

NH₂N(R¹)₂ (Vb) or

NH₂N(R¹)CON(R¹)₂ (Vc)

wherein R¹ is as hereinbefore defined or with an acid addition salt (preferably the hydrochloride) thereof.

The reaction is generally carried out in the presence of an inorganic base, e.g. sodium carbonate or sodium acetate in an inert organic solvent, e.g. ethanol, or an organic base, e.g. pyridine, which may serve as the solvent, in an inert organic solvent at a temperature from 0°C to 120°C.

A stereoselective synthesis may be performed in which a mixture of enantiomers of general formula (III) wherein X′ represents the carbonyl group is reacted with a chiral auxiliary agent, e.g. (S)-1-amino-2-methoxymethylpyrrolidine, before being reacted with a compound of general formula (IV) as hereinbefore described followed by the removal of the chiral auxiliary agent.

According to a feature of the invention, the thioformamide derivatives of general formula (I) wherein R represents the methyl radical and X represents the carbonyl group or a group of the formula >C=NOR¹ or >C=NN(R¹)₂ as hereinbefore defined may be prepared by the process which comprises reacting methylamine with a dithioester of the general formula (VI) wherein the symbols Ar, X′ and Y are as hereinbefore defined, and R′ represents a straight- or branched-chain alkyl radical containing 1 to 4 carbon atoms, or a benzyl or carboxymethyl radical.

In general, the reaction is carried out with an excess of methylamine, without a solvent or in an organic solvent such as an aromatic hydrocarbon, an ether or an alcohol of low molecular weight, or a mixture of these solvents, at a temperature from 20° to 130°C, optionally under pressure.

It is particularly advantageous for the thiol formed during the reaction to be fixed in the form of a heavy metal salt using a thiol acceptor such as mercuric chloride.

The dithioester of general formula (VI) can be obtained by the following methods:
(1) By reaction of a strong base with a compound of the general formula (III) (wherein X′, Ar, and Y are as hereinbefore defined), followed by reacting the resulting product with carbon disulphide and then with a compound of the general formula:

   R′-Z (VII)

   wherein R′ is as hereinbefore defined, and Z represents a halogen atom, preferably a chlorine, bromine or iodine atom, or a reactive ester radical, preferably a mesyloxy or tosyloxy radical.

The reaction is generally carried out in an ether such as tetrahydrofuran, to which hexamethylphosphoramide has generally been added, at a temperature between -20° and +50°C.

It is particularly advantageous to employ potassium tert.-butoxide as the strong base. Alternatively the organo-lithium derivatives described above may be employed.

It will be understood that it may be desirable to change one or more of the substituents at an appropriate stage during the synthesis of the compounds of the invention, for example, the compounds of general formula (I) wherein Ar represents a phenyl group substituted by a carbamoyl group may be alternatively prepared from the corresponding compounds of general formula (I) wherein Ar represents a phenyl group substituted by a cyano group by the application or adaptation of known methods for such conversion.

The thioformamide derivatives of general formula (I) obtained by the aforedescribed processes can be purified by the usual physical methods, in particular crystallisation and chromatography, especially to resolve mixtures of enantiomers using a chiral column.

Compounds of general formulae (III) and (V) may be prepared by known methods.

By the term "pharmaceutically acceptable salts" as used in this specification is meant salts the anions or cations of which are relatively innocuous to the animal organism when used in therapeutic doses so that the beneficial pharmaceutical properties of the parent compounds of general formula (I) capable of forming salts are not vitiated by side-effects ascribable to those anions or cations.

As well as being useful in themselves as active compounds, acid addition salts of the compounds of general formula (I) capable of forming such salts are useful for the purposes of purification of the parent compounds of general formula (I), for example by exploitation of the solubility differences between the salts and the parent compounds, by techniques well known to those skilled in art. The parent compounds of general formula (I) can be regenerated from their acid addition salts by known methods, for example by treatment with an alkali, e.g. aqueous sodium bicarbonate solution or aqueous ammonia solution.

Suitable acid addition salts for use in pharmaceuticals may be selected from salts derived from inorganic acids, for example hydrochlorides, hydrobromides, phosphates, sulphates and nitrates, and organic acids, for example oxalates, lactates, tartrates, acetates, salicylates, citrates, propionates, succinates, fumarates, maleates, methylene-bis-β-hydroxynaphthoates, gentisates and di-p-toluoyltartrates.

As well as being useful in themselves as active compounds, salts of the compounds of general formula (I) capable of forming salts with bases are useful for the purposes of purification of the parent compounds of general formula (I), for example by exploitation of the solubility differences between the salts and the parent compounds, by techniques well known to those skilled in the art.

Suitable salts with bases include alkali metal (e.g. sodium and potassium), alkaline earth metal (e.g. calcium and magnesium), ammonium and amine (e.g. diethanolamine, triethanolamine, octylamine, morpholine and dioctylmethylamine) salts.

The following Examples illustrate the preparation of compounds according to the present invention.

Unless stated otherwise, all the spectra were recorded at 200 MHz in deuterochloroform; the chemical shifts are expressed in ppm relative to the tetramethylsilane signal. The abbreviations used in the following text are as follows:
- s =: singlet
- d =: doublet
- t =: triplet
- m =: multiplet
- c =: unresolved bands
- dd =: doublet of doublets
- dt =: doublet of triplets
- ddd =: doublet of doublets of doublets
- dddd =: doublet of doublets of doublets of doublets

### EXAMPLE 1

### Compounds A, E and F

A solution of (±)-2-phenylcyclohexanone (3.0g, 17.2 mmol) at -15°C in tetrahydrofuran (40 ml) was treated with potassium t.-butoxide (1.9g, 17.2 mmol) during 2 minutes. After 15 minutes at -15°C, a solution of methyl isothiocyanate (1.38g, 19 mmol) in tetrahydrofuran (20 ml) was added dropwise during 2 minutes and the resulting solution was stirred for 3 hours at 0°C. Water (200 ml) followed by chloroform (200 ml) were added to the reaction mixture and the aqueous layer was extracted with chloroform (200 ml). The combined organic extracts were washed with brine (150 ml) then dried over sodium sulphate. Concentration in vacuo (30°C; 1.9 kPa (14 mm Hg)) afforded a crude oil (4.2g) which was purified by flash chromatography over silica gel eluting with a 1:1 mixture of diethyl ether and hexane to give (±)-N-methyl-2-oxo-1-phenylcyclohexanecarbothioamide (1.5g, 6.1 mmol) m.p. 86°C. N.M.R. (CDCl₃) 1.62 - 2.05 (c, 4H), 2.42 - 2.56 (m, 2H), 2.64 - 2.84 (ddd, 1H), 3.0 - 3.18 (c, 4H), 7.23 - 7.45 (c, 5H) 8.72 - 9.06 (broad singlet, 1H). Found C, 68.3; H, 7.2; N, 5.8; S, 13.0%, C₁₄H₁₇NOS requires C, 68.0; H, 6.9; N, 5.7; S, 13.0%.

By proceeding in a similar manner to that hereinbefore described but replacing the (±)-2-phenylcyclohexanone by the appropriately substituted (±)-2-phenylcyclohexanone, there were prepared:-
(±)-N-methyl-2-oxo-1-(4-chlorophenyl)cyclohexanecarbothioamide, a colourless solid, melting point 125-127°C, after being purified by flash chromatography on silica gel eluting with toluene/ethyl acetate : 100/0.25, followed by trituration with ether;
(±)-N-methyl-2-oxo-1-(3-trifluoromethylphenyl)cyclohexanecarbothioamide, a pale yellow solid, melting point 118-120°C, after purification by flash chromatography on silica gel eluting with ether/pentane : 1/1, followed by trituration with pentane. The reaction was carried out at -40°C. After addition of methyl isothiocyanate the temperature was allowed to rise slowly to 20°C and maintained at that level for 24 hours.

### EXAMPLE 2

### Compound B

A solution of (±)-N-methyl-2-oxo-1-phenylcyclohexanecarbothioamide (0.44g, 1.78 mmol) in a mixture of dimethylsulphoxide (6 ml) and methanol (6 ml) at -10°C was treated with sodium borohydride (0.07g, 1.8 mmol) in one portion. After 2 hours at -10°C the reaction mixture was warmed at 20°C and maintained at this temperature for 1.5 hours. The mixture was treated with water (20 ml) then extracted with ethyl acetate (2 x 50 ml). The combined extracts were washed with brine then dried over sodium sulphate and concentrated in vacuo to give a crude oil (0.58g) which was recrystallised from a 3:1 mixture of hexane and ethyl acetate to give (±)-trans-N-methyl-2-hydroxy-1-phenylcyclohexanecarbothioamide (0.2g, 8.0mmol) m.p. 118-119°C. N.M.R. (CDCl₃) 1.2 - 1.78 (m, 6H), 1.80 - 2.0 (m, 1H), 2.14 - 2.30 (dt, 1H), 2.30 - 2.46 (ddd, 1H), 3.0 - 3.08 (d, 3H), 4.76 - 4.94 (m, 1H), 7.0 - 7.24 (broad singlet, 1H), 7.24 - 7.48 (m, 3H), 7.64 - 7.84 (m, 2H). Found: C, 67.9; H, 7.6; N, 5.5; S, 12.9%: C₁₄H₁₉NOS requires C, 67.4; H, 7.7; N, 5.6; S, 12.9%.

### EXAMPLE 3

### Compound C

A suspension of (±)-N-methyl-2-oxo-1-phenylcyclohexanecarbothioamide (0.6g, 2.4 mmol) in pyridine (6 ml) at 20°C was treated with hydroxylamine hydrochloride (0.34g, 4.9 mmol) and stirred for 18 hours. The resulting solution was poured into water (50 ml) and extracted with ethyl acetate (3 x 50 ml). The combined organic extracts were washed successively with water (15 ml) and brine (15 ml) then dried over sodium sulphate. Concentration in vacuo (30°C; 1.9 kPa (14mm Hg)) afforded a crude oil which was recrystallised from isopropanol (6 ml) to give (±)-anti-N-methyl-2-hydroxyimino-1-phenylcyclohexanecarbothioamide (0.27g, 1.03 mmol) m.p. 182-184°C. N.M.R. (CDCl₃) 1.28 - 1.84 (c, 4H); 2.20 - 2.40 (m, 1H); 2.42 - 2.58 (m, 1H); 2.78 - 2.86 (dt, 1H); 3.04 - 3.26 (c, 4H); 7.20 - 7.58 (m, 6H); 8.40 (s, 1H); Found: C, 64.4; H, 7.0; N, 10.6; S, 12.1% C₁₄H₁₈N₂OS requires C, 64.1; H, 6.9; N, 10.7; S, 12.2%.

### EXAMPLE 4

### Compound D

A suspension of (±)-N-methyl-2-oxo-1-phenylcyclohexanethioamide (0.5 g, 2 mmol), in pyridine (5 ml) at 20°C was treated with O-methylhydroxylamine hydrochloride (0.34 g, 4 mmol).

After stirring for 48 hours at 20°C the solution was poured into water (50 ml) and the mixture was then extracted with ethyl acetate (4 x 50 ml). The combined organic extracts were washed with water (20 ml) then dried over sodium sulphate. Concentration in vacuo (25°C 1.9 kPa (14 mmHg)) afforded a crude oil (0.67 g) which was recrystallised from isopropanol to give (±)-N-anti-N-methyl-2-methoxyimino-1-phenylcyclohexanecarbothioamide (0.2 g, 0.7 mml), melting point 79-80°C. N.M.R. (CDCl₃) 1.34-1.72 (c, 3H), 2.72-2.92 (m, 1H), 2.16-2.36 (m, 1H), 2.56-2.96 (m, 3H), 3.10-3.16 (d, 3H), 3.86 (s, 3H), 7.20-7.46 (c, 5H), 8.10-8.44 (broad singlet, 1H). Found C, 65.2; H, 7.6; N, 10.2; S, 11.7% : C₁₅H₂₀N₂OS. Requires C, 65.2; H, 7.3; N, 10.1; S, 11.6%.

### EXAMPLE 5

### Compounds G and H

A stirred suspension of (±)-N-methyl-2-oxo-1-(3,4-dichlorophenyl)cyclohexanecarbothioamide (2.75g) and benzyloxyamine hydrochloride (1.46g) in ethanol (25ml) and pyridine (5ml) was refluxed for 24 hours. The mixture was evaporated, the residue dissolved in chloroform (75ml), washed with water (3x50ml), dried over magnesium sulphate, and evaporated. The residual oil was purified by flash chromatography on silica, eluting with toluene to give (±)-anti-N-methyl-2-benzyloxyimino-1-(3,4-dichlorophenyl)cyclohexanecarbothioamide (2.8g), yellow crystals, m.p. 103-105°C.

By proceeding in a similar manner to that hereinbefore described but replacing the (±)-N-methyl-2-oxo-1-(3,4-dichlorophenyl) cyclohexanecarbothioamide by (±)-N-methyl-2-oxo-1-phenylcyclohexanecarbothioamide and benzyloxyamine hydrochloride by 4-fluorobenzyloxyamine hydrochloride, there was prepared:-
(±)-anti-N-methyl-2-(4-fluorobenzyloxyimino)-1-phenylcyclohexanecarbothioamide, m.p. 65°C.

### EXAMPLE 6

### Compounds I and J

By proceeding in a similar manner to that hereinbefore described in Example 1, there were prepared:-
(±)-N-methyl-2-oxo-1-(2-naphthyl)cyclohexanecarbothioamide, a colourless crystalline solid, m.p. 126-127°C, after purification by flash chromatography over silica gel eluting with toluene/ethyl acetate: 99/1 and triturating with ether;
(±)-N-methyl-2-oxo-1-(3,4-dichlorophenyl)cyclohexanecarbothioamide, a colourless crystalline solid, m.p. 146-147°C, after purification by flash chromatography on silica gel eluting with toluene and triturating with ether.

### EXAMPLE 7

### Compound K

A solution of (±)-2-(3-nitrophenyl)cyclohexanone (1g,4.5mmol) and methyl isothiocyanate at 20°C in tetrahydrofuran (15ml) was treated with sodium hydride (120mg, 5mmol) added in one portion. The mixture was stirred vigorously. After 13 minutes, water (20ml) was added and the mixture extracted with ethyl acetate (2x20ml). The combined organic extracts were dried over magnesium sulphate. Concentration in vacuo (30°C,1.9 kPa (14mmHg)) afforded a crude oil (0.95g) which was purified by flash chromatography over silica gel, eluting with toluene/acetone: 96/4 and trituration with equal portions of hexane and diethyl ether to give (±)-N-methyl-2-oxo-1-(3-nitrophenyl)cyclohexanecarbothioamide (0.1g, 0.35mmol) m.p. 128-132°C.

The present invention includes within its scope pharmaceutical compositions which comprise a compound of general formula (I) or a pharmaceutically acceptable salt thereof, in association with a pharmaceutically acceptable carrier or coating. In clinical practice the compounds of the present invention may be administered rectally, but are preferably administered parenterally, by inhalation if appropriate, or, more preferably, orally.

Solid compositions for oral administration include compressed tablets, pills, powders and granules. In such solid compositions, one or more of the active compounds is, or are, admixed with at least one inert diluent such as starch, sucrose or lactose. The compositions may also comprise, as is normal practice, additional substances other than inert diluents, e.g. lubricating agents, such as magnesium stearate.

Liquid compositions for oral administration include pharmaceutically acceptable emulsions, solutions, suspensions, syrups and elixirs containing inert diluents commonly used in the art such as water and liquid paraffin. Besides inert diluents such compositions may comprise adjuvants, such as wetting, and suspending agents, and sweetening, flavouring, perfuming and preserving agents. The compositions according to the invention for oral administration also include capsules of absorbable material such as gelatin, containing one or more of the active substances with or without the addition of diluents or excipients.

Preparations according to the invention for parenteral administration include sterile aqueous, aqueous-organic, and organic solutions, suspensions and emulsions. Examples of organic solvents or suspensing media are propylene glycol, polyethylene glycol, vegetable oils such as olive oil and injectable organic esters such as ethyl oleate. The compositions may also contain adjuvants such as stabilising, preserving, wetting, emulsifying and dispersing agents. They may be sterilized by, for example, filtration through a bacteria-retaining filter, by incorporation in the compositions of sterilizing agents, by irradiation or by heating. They may also be manufactured in the form of sterile solid compositions, which can be dissolved in sterile water or some other sterile injectable medium immediately before use.

Compositions for inhalation may be sterile aqueous solutions which are then nebulised or dry powders formulated in accordance with known methods.

Solid compositions for rectal administration include suppositories formulated in accordance with known methods and containing one or more of the compounds of formula (I) or a pharmaceutically acceptable salt thereof.

The percentage of active ingredient in the compositions of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration and the duration of the treatment, and the condition of the patient. In the adult, the doses are generally from 0.001 to 50 mg/kg body weight per day by oral administration. By inhalation, either as a nebulised solution or as a formulated dry powder, the preferred daily dosage is from 0.001 to 5 mg/kg body weight. The compounds may also be applied topically for inhibition of head hair loss associated with male pattern baldness, the preferred daily dosage being from 0.1 to 10 mg/kg body weight applied, for example, in 5ml portions two or three times per day.

The following Example illustrates pharmaceutical compositions according to the present invention.

### COMPOSITION EXAMPLE

No. 2 size gelatin capsules each containing:-

| | |
|---|---|
| (±)-N-methyl-2-oxo-1-phenylcyclohexanecarbothiamide | 20 mg |
| lactose | 100 mg |
| starch | 60 mg |
| dextrin | 40 mg |
| magnesium stearate | 1 mg |

were prepared in accordance with the usual procedure.

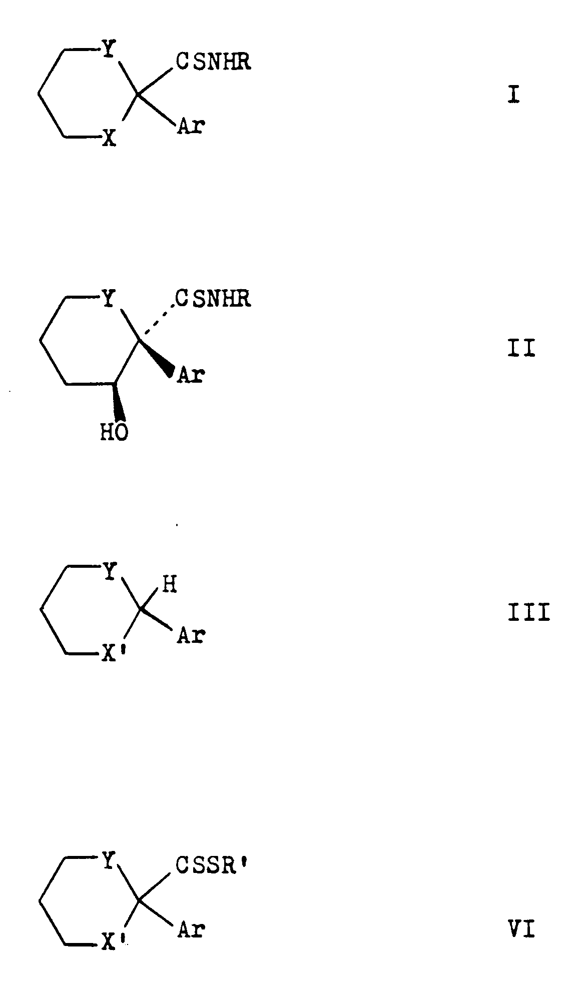

## Claims (Claims for the following Contracting State(s): AT, BE, CH, DE, FR, GB, IT, LI, LU, NL, SE)

1. A thioformamide derivative of the general formula: 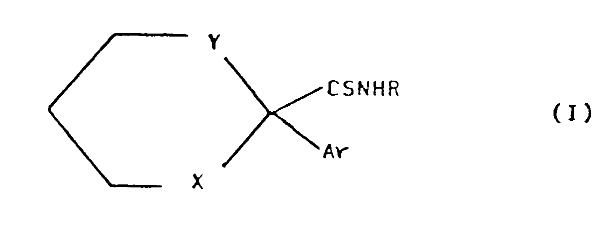 wherein R represents a straight- or branched-chain alkyl radical containing from 1 to 4 carbon atoms, Ar represents a phenyl group unsubstituted or substituted in the 3 and/or 5 position with an electron-withdrawing group selected from a cyano, nitro, trifluoromethyl, carbamoyl, carboxy, C₂₋₅-alkanoyl, C₂₋₅-alkoxycarbonyl or C₁₋₄-alkylsulphonyl group or a fluorine, chlorine or bromine atom, and optionally further substituted with halogen atom(s), C₁₋₄-alkyl or C₆₋₁₂-aryl group(s), or the group Ar is substituted with halogen atom(s), C₁₋₄-alkyl or C₆₋₁₂-aryl group(s) or with substituents which together form a fused ring, Y represents an ethylene or methylene radical or a valency bond, and X represents a carbonyl or hydroxymethylene group or a group of the formula: >C=NOR¹, >C=NN(R¹)₂ or >C=NN(R¹)CON(R¹)₂ in which the symbols R¹, which may be the same or different, each represents the hydrogen atom or a straight- or branched-chain alkyl radical containing from 1 to 4 carbon atoms which is unsubstituted or substituted by one or more substituents selected from C₂₋₄-alkenyl, carboxy, C₂₋₅-alkoxycarbonyl, hydroxy, C₁₋₄-alkoxy, carbamoyl (unsubstituted or substituted by one or two C₁₋₄-alkyl groups), amino, C₁₋₄-alkylamino and di-C₁₋₄-alkylamino groups, or represents a benzyl, phenethyl, 1-naphthylmethyl, 2-naphthylmethyl or pyrid-3-ylmethyl radical each of which may be substituted on the ring by one or more halogen atoms or hydroxy, C₁₋₄-alkyl, C₁₋₄-alkoxy (alkoxy being unsubstituted or substituted as defined for alkyl groups represented by R¹), cyano, nitro, trifluoromethyl, carboxy, C₁₋₄-alkylamino, C₂₋₅-alkanoylamino or C₂₋₅-alkoxycarbonyl groups or two R¹ substituents on the same nitrogen atom may together form a straight- or branched-chain alkylene radical containing from 4 to 6 carbon atoms in the chain which is unsubstituted or substituted as defined for alkyl radicals represented by R¹, and pharmaceutically acceptable salts thereof.

2. A compound according to claim 1 wherein X represents the carbonyl group or a group of formula >C=NOR¹ as defined in claim 1.

3. A compound according to claim 1 or 2 wherein Y represents a methylene radical.

4. A compound according to claim 1, 2 or 3 wherein Ar represents the phenyl, 4-chlorophenyl, 3-trifluoromethylphenyl, 3-nitrophenyl, 3,4-dichlorophenyl or 2-naphthyl group.

5. A compound according to any one of claims 1, 3 and 4 of the general formula: 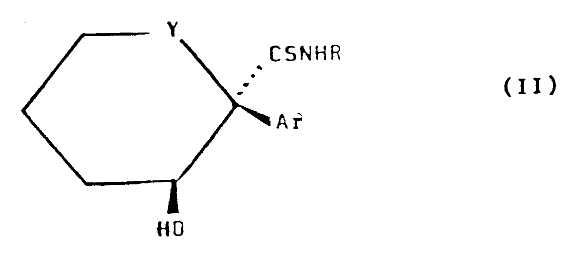 (wherein Y, R and Ar are as defined in any one of the preceding claims) in which the hydroxy group is in the trans position relative to the group -CSNHR.

6. A compound according to claim 1 which is:
(±)-N-methyl-2-oxo-1-phenylcyclohexanecarbothioamide,
(±)-trans-N-methyl-2-hydroxy-1-phenylcyclohexanecarbothioamide,
(±)-anti-N-methyl-2-hydroxyimino-1-phenylcyclohexanecarbothioamide,
(±)-anti-N-methyl-2-methoxyimino-1-phenylcyclohexanecarbothioamide,
(±)-N-methyl-2-oxo-1-(4-chlorophenyl)cyclohexanecarbothioamide,
(±)-N-methyl-2-oxo-1-(3-trifluoromethylphenyl)-cyclohexanecarbothioamide,
(±)-anti-N-methyl-2-benzyloxyimino-1-(3,4-dichlorophenyl)-cyclohexanecarbothioamide,
(±)-anti-N-methyl-2-(4-fluorobenzyloxyimino)-1-phenylcyclohexanecarbothioamide,
(±)-N-methyl-2-oxo-1-(2-naphthyl)-cyclohexanecarbothioamide,
(±)-N-methyl-2-oxo-1-(3,4-dichlorophenyl)-cyclohexanecarbothioamide, or
(±)-N-methyl-2-oxo-1-(3-nitrophenyl)-cyclohexanecarbothioamide,
or a pharmaceutically acceptable salt thereof.

7. A process for the preparation of a thioformamide derivative of general formula (I) depicted in claim 1 which comprises:-
(A) when X represents the carbonyl group or a group of formula >C=NOR¹ or >C=NN(R¹)₂ wherein R¹ is as defined in claim 1 and Y, R and Ar are as defined in claim 1 the reaction of a compound of the general formula: 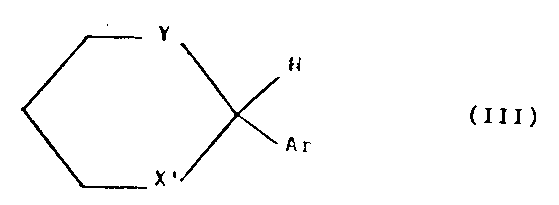 wherein X' represents the carbonyl group or a group of formula >C=NOR¹ or >C=NN(R¹)₂ as defined in claim 1 and Y and Ar are as defined in claim 1 with an isothiocyanate of the general formula:
R-N=C=S (IV)
wherein R represents a straight- or branched-chain alkyl radical containing 1 to 4 carbon atoms;
(B) when X represents the carbonyl group the stereoselective reaction of a mixture of enantiomers of general formula (III) hereinbefore depicted wherein Y and Ar are as defined in claim 1 and X' represents the carbonyl group with a chiral auxiliary agent and reaction of the product obtained with a compound of the general formula:
R-N=C=S (IV)
wherein R is as defined in claim 1 followed by removal of the chiral auxiliary agent;
(C) when X represents the hydroxymethylene group and Y, R and Ar are as defined in claim 1 the reduction of a compound of general formula (I) wherein X represents the carbonyl group and Y, R and Ar are as defined in claim 1;
(D) when X represents a group of the formula >C=NOR¹, >C=NN(R¹)₂ or >C=NN(R¹)CON(R¹)₂ as defined in claim 1 and Y, R and Ar are as defined in claim 1 the reaction of a compound of general formula (I) wherein X represents the carbonyl group with a compound of general formula:
NH₂OR¹ (Va)
NH₂N(R¹)₂ (Vb) or
NH₂N(R¹)CON(R¹)₂ (Vc)
wherein R¹ is as defined in claim 1 or with an acid addition salt thereof;
(E) when R represents the methyl radical and X represents the carbonyl group or a group of the formula >C=NOR¹ or >C=NN(R¹)₂ as defined in claim 1, and Y and Ar are as defined in claim 1 the reaction of methylamine with a dithioester of the general formula: 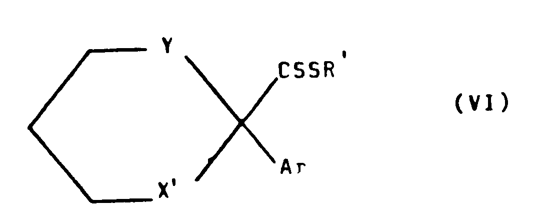 wherein R′ represents a straight- or branched-chain alkyl radical containing 1 to 4 carbon atoms, or a benzyl or carboxymethyl radical, X′ is as hereinbefore defined and Y and Ar are as defined in claim 1;
optionally followed by the step of converting a thioformamide derivative thus obtained into a pharmaceutically acceptable salt thereof.

8. A pharmaceutical composition which comprises a thioformamide derivative of general formula (I) as defined in claim 1 or a pharmaceutically acceptable salt thereof in association with a pharmaceutically acceptable carrier or coating.

## Claims (Claims for the following Contracting State(s): ES, GR)

1. A process for the preparation of a thioformamide derivative of the general formula: 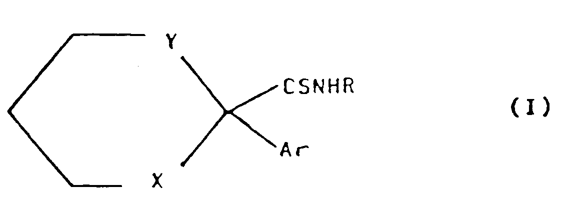 wherein R represents a straight- or branched-chain alkyl radical containing from 1 to 4 carbon atoms, Ar represents a phenyl group unsubstituted or substituted in the 3 and/or 5 position with an electron-withdrawing group selected from a cyano, nitro, trifluoromethyl, carbamoyl, carboxy, C₂₋₅-alkanoyl, C₂₋₅-alkoxycarbonyl or C₁₋₄-alkylsulphonyl group or a fluorine, chlorine or bromine atom, and optionally further substituted with halogen atom(s), C₁₋₄-alkyl or C₆₋₁₂-aryl group(s), or the group Ar is substituted with halogen atom(s), C₁₋₄-alkyl or C₆₋₁₂-aryl group(s) or with substituents which together form a fused ring, Y represents an ethylene or methylene radical or a valency bond, and X represents a carbonyl or hydroxymethylene group or a group of the formula: >C=NOR¹, >C=NN(R¹)₂ or >C=NN(R¹)CON(R¹)₂ in which the symbols R¹, which may be the same or different, each represents the hydrogen atom or a straight- or branched-chain alkyl radical containing from 1 to 4 carbon atoms which is unsubstituted or substituted by one or more substituents selected from C₂₋₄-alkenyl, carboxy, C₂₋₅-alkoxycarbonyl, hydroxy, C₁₋₄-alkoxy, carbamoyl (unsubstituted or substituted by one or two C₁₋₄-alkyl groups), amino, C₁₋₄-alkylamino and di-C₁₋₄-alkylamino groups, or represents a benzyl, phenethyl, 1-naphthylmethyl, 2-naphthylmethyl or pyrid-3-ylmethyl radical each of which may be substituted on the ring by one or more halogen atoms or hydroxy, C₁₋₄-alkyl, C₁₋₄-alkoxy (alkoxy being unsubstituted or substituted as defined for alkyl groups represented by R¹), cyano, nitro, trifluoromethyl, carboxy, C₁₋₄-alkylamino, C₂₋₅-alkanoylamino or C₂₋₅-alkoxycarbonyl groups or two R¹ substituents on the same nitrogen atom may together form a straight- or branched-chain alkylene radical containing from 4 to 6 carbon atoms in the chain which is unsubstituted or substituted as defined for alkyl radicals represented by R¹, and pharmaceutically acceptable salts thereof; which process comprises:-
(A) when X represents the carbonyl group or a group of formula >C=NOR¹ or >C=NN(R¹)₂ wherein R¹ is as hereinbefore defined and Y, R and Ar are as hereinbefore defined the reaction of a compound of the general formula: 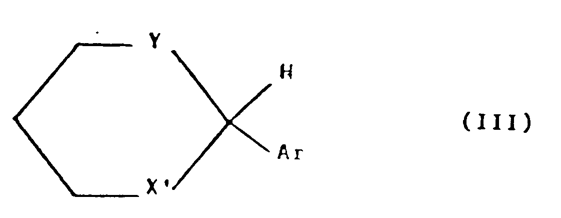 wherein X' represents the carbonyl group or a group of formula >C=NOR¹ or >C=NN(R¹)₂ as hereinbefore defined and Y and Ar are as hereinbefore defined with an isothiocyanate of the general formula:
R-N=C=S (IV)
wherein R represents a straight- or branched-chain alkyl radical containing 1 to 4 carbon atoms;
(B) when X represents the carbonyl group the stereoselective reaction of a mixture of enantiomers of general formula (III) hereinbefore depicted wherein Y and Ar are as hereinbefore defined and X' represents the carbonyl group with a chiral auxiliary agent and reaction of the product obtained with a compound of the general formula:
R-N=C=S (IV)
wherein R is as hereinbefore defined followed by removal of the chiral auxiliary agent;
(C) when X represents the hydroxymethylene group and Y, R and Ar are as hereinbefore defined the reduction of a compound of general formula (I) wherein X represents the carbonyl group and Y, R and Ar are as hereinbefore defined;
(D) when X represents a group of the formula >C=NOR¹, >C=NN(R¹)₂ or >C=NN(R¹)CON(R¹)₂ as hereinbefore defined and Y, R and Ar are as hereinbefore defined the reaction of a compound of general formula (I) wherein X represents the carbonyl group with a compound of general formula:
NH₂OR¹ (Va)
NH₂N(R¹)₂ (Vb) or
NH₂N(R¹)CON(R¹)₂ (Vc)
wherein R¹ is as hereinbefore defined or with an acid addition salt thereof;
(E) when R represents the methyl radical and X represents the carbonyl group or a group of the formula >C=NOR¹ or >C=NN(R¹)₂ as hereinbefore defined, and Y and Ar are as hereinbefore defined the reaction of methylamine with a dithioester of the general formula: 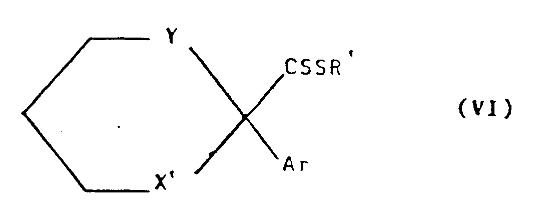 wherein R' represents a straight- or branched-chain alkyl radical containing 1 to 4 carbon atoms, or a benzyl or carboxymethyl radical, X' is as hereinbefore defined and Y and Ar are as hereinbefore defined;
optionally followed by the step of converting a thioformamide derivative thus obtained into a pharmaceutically acceptable salt thereof.

2. A process according to claim 1 for the preparation of a compound of the general formula (I) wherein X represents the carbonyl group or a group of formula >C=NOR¹ as defined in claim 1.

3. A process according to claim 1 or 2 for the preparation of a compound of the general formula (I) wherein Y represents a methylene radical.

4. A process according to claim 1, 2 or 3 for the preparation of a compound of the general formula (I) wherein Ar represents the phenyl, 4-chlorophenyl, 3-trifluoromethylphenyl, 3-nitrophenyl, 3,4-dichlorophenyl or 2-naphthyl group.

5. A process according to any one of claims 1, 3 and 4 for the preparation of a compound of the general formula : 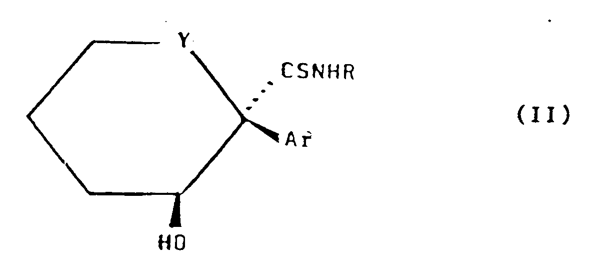 (wherein Y, R and Ar are as defined in any one of the preceding claims) in which the hydroxy group is in the trans position relative to the group -CSNHR.

6. A process according to claim 1 for the preparation of a compound of the general formula (I) which is:
(±)-N-methyl-2-oxo-1-phenylcyclohexanecarbothioamide,
(±)-trans-N-methyl-2-hydroxy-1-phenylcyclohexanecarbothioamide,
(±)-anti-N-methyl-2-hydroxyimino-1-phenylcyclohexanecarbothioamide,
(±)-anti-N-methyl-2-methoxyimino-1-phenylcyclohexanecarbothioamide,
(±)-N-methyl-2-oxo-1-(4-chlorophenyl)cyclohexanecarbothioamide,
(±)-N-methyl-2-oxo-1-(3-trifluoromethylphenyl)-cyclohexanecarbothioamide,
(±)-anti-N-methyl-2-benzyloxyimino-1-(3,4-dichlorophenyl)-cyclohexanecarbothioamide,
(±)-anti-N-methyl-2-(4-fluorobenzyloxyimino)-1-phenylcyclohexanecarbothioamide,
(±)-N-methyl-2-oxo-1-(2-naphthyl)-cyclohexanecarbothioamide,
(±)-N-methyl-2-oxo-1-(3,4-dichlorophenyl)-cyclohexanecarbothioamide, or
(±)-N-methyl-2-oxo-1-(3-nitrophenyl)-cyclohexanecarbothioamide,
or a pharmaceutically acceptable salt thereof.

7. A process according to any one of the preceding claims followed by the step of formulating a thioformamide derivative of general formula (I) defined in claim 1 or a pharmaceutically acceptable salt thereof with a pharmaceutically acceptable carrier or coating.

## Patentansprüche (Patentansprüche für folgende(n) Vertragsstaat(en): AT, BE, CH, DE, FR, GB, IT, LI, LU, NL, SE)

1. Thioformamidderivat der allgemeinen Formel: 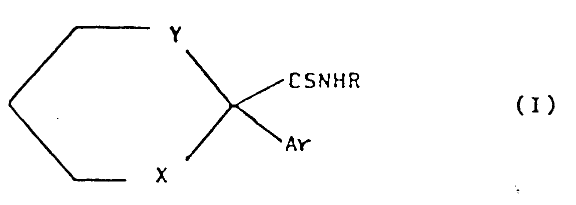 worin R für einen gerad- oder verzweigtkettigen Alkylrest mit 1 bis 4 Kohlenstoffatom(en) steht, Ar eine unsubstituierte oder in 3- und/oder 5-Stellung(en) mit einer elektronenziehenden Gruppe, ausgewählt aus einer Cyano-, Nitro-, Trifluormethyl-, Carbamoyl-, Carboxy-, C₂₋₅-Alkanoyl-, C₂₋₅-Alkoxycarbonyl- oder C₁₋₄-Alkylsulfonylgruppe oder einem Fluor-, Chlor- oder Bromatom, und gegebenenfalls weiter durch (ein) Halogenatom(e) oder (eine) C₁₋₄-Alkyl- oder C₆₋₁₂-Arylgruppe(n) substituierte Phenylgruppe bedeutet, oder die Gruppe Ar durch (ein) Halogenatom(e), (eine) C₁₋₄-Alkyl- oder C₆₋₁₂-Arylgruppe(n) oder durch zusammen einen ankondensierten Ring bildende Substituenten substituiert ist, Y einem Ethylen- oder Methylenrest oder einer Valenzbindung entspricht und X eine Carbonyl- oder Hydroxymethylengruppe oder eine Gruppe der Formeln: >C=NOR¹, >C=NN(R¹)₂ oder >C-NN(R¹)CON(R¹)₂, worin die Symbole R¹, die gleich oder verschieden sein können, jeweils für das Wasserstoffatom oder einen gerad- oder verzweigtkettigen Alkylrest mit 1 bis 4 Kohlenstoffatom(en), der unsubstituiert oder durch einen oder mehrere Substituenten, ausgewählt aus C₂₋₄-Alkenyl-, Carboxy-, C₂₋₅-Alkoxycarbonyl-, Hydroxy-, C₁₋₄-Alkoxy-, Carbamoyl-(unsubstituiert oder durch C₁₋₄-Alkylgruppen ein- oder zweifach substituiert), Amino-, C₁₋₄-Alkylamino- und Di-C₁₋₄-alkylaminogruppen, substituiert ist, oder einen Benzyl-, Phenethyl-, 1-Naphthylmethyl-, 2-Naphthylmethyl- oder Pyrid-3-ylmethylrest, von denen jeder am Ring durch ein(e) oder mehrere Halogenatom(e) oder Hydroxy-, C₁₋₄-Alkyl-, C₁₋₄-Alkoxy-(die Alkoxygruppe ist unsubstituiert oder in der für die durch R¹ darstellbaren Alkylgruppen angegebenen Weise substituiert), Cyano-, Nitro-, Trifluormethyl-, Carboxy-, C₁₋₄-Alkylamino-, C₂₋₅-Alkanoylamino- oder C₂₋₅-Alkoxycarbonylgruppen substituiert sein kann, stehen oder zwei R¹-Substituenten am selben Stickstoffatom zusammen einen gerad- oder verzweigtkettigen Alkylenrest mit 4 bis 6 Kohlenstoffatomen in der Kette, die unsubstituiert oder in der für die durch R¹ darstellbaren Alkylreste angegebenen Weise substituiert ist, bilden können, bedeutet;
oder pharmazeutisch akzeptable Salze desselben.

2. Verbindung nach Anspruch 1, worin X für die Carbonylgruppe oder eine Gruppe der Formel >C=NOR¹ in der Definition gemäß Anspruch 1 darstellt.

3. Verbindung nach Anspruch 1 oder 2, worin Y für einen Methylenrest steht.

4. Verbindung nach Anspruch 1, 2 oder 3, worin Ar für die Phenyl-, 4-Chlorphenyl-, 3-Trifluormethylphenyl-, 3-Nitrophenyl-, 3,4-Dichlorphenyl- oder 2-Naphthylgruppe steht.

5. Verbindung nach einem der Ansprüche 1, 3 und 4 der allgemeinen Formel: 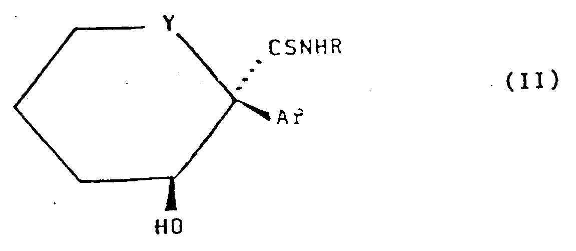 mit Y, R und Ar in der Definition gemäß einem der vorhergehenden Ansprüche, worin sich die Hydroxygruppe in trans-Stellung bezüglich der Gruppe -CSNHR befindet.

6. Verbindung nach Anspruch 1, nämlich:
(±)-N-Methyl-2-oxo-1-phenylcyclohexancarbothioamid,
(±)-trans-N-Methyl-2-hydroxy-1-phenylcyclohexancarbothioamid,
(±)-anti-N-Methyl-2-hydroxyimino-1-phenylcyclohexancarbothioamid,
(±)-anti-N-Methyl-2-methoxyimino-1-phenylcyclohexancarbothioamid,
(±)-N-Methyl-2-oxo-1-(4-chlorphenyl)-cyclohexancarbothioamid,
(±)-N-Methyl-2-oxo-1-(3-trifluormethylphenyl)-cyclohexancarbothioamid,
(±)-anti-N-Methyl-2-benzyloxyimino-1-(3,4-dichlorphenyl)-cyclohexancarbothioamid,
(±)-anti-N-Methyl-2-(4-fluorbenzyloxyimino)-1-phenylcyclohexancarbothioamid,
(±)-N-Methyl-2-oxo-1-(2-naphthyl)-cyclohexancarbothioamid,
(±)-N-Methyl-2-oxo-1-(3,4-dichlorphenyl)-cyclohexancarbothioamid oder
(±)-N-Methyl-2-oxo-1-(3-nitrophenyl)-cyclohexancarbothioamid,
oder ein pharmazeutisch akzeptables Salz desselben.

7. Verfahren zur Herstellung eines Thioformamidderivats der allgemeinen Formel (I) gemäß Anspruch 1 durch
(A) - wenn X für die Carbonylgruppe oder eine Gruppe der Formel >C=NOR¹ oder >C=NN(R¹)₂ mit R¹ in der Definition gemäß Anspruch 1 steht und Y, R und Ar die in Anspruch 1 angegebene Bedeutung besitzen - Umsetzen einer Verbindung der allgemeinen Formel: 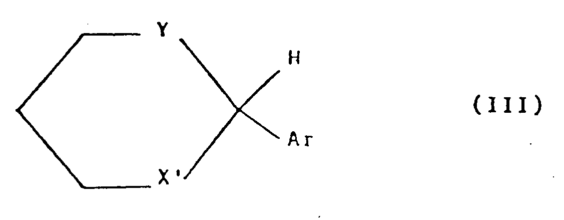 worin X' für die Carbonylgruppe oder eine Gruppe der Formel >C=NOR¹ oder >C=NN(R¹)₂ entsprechend der Definition in Anspruch 1 steht und Y und Ar die in Anspruch 1 angegebene Bedeutung besitzen, mit einem Isothiocyanat der allgemeinen Formel:
R-N=C=S (IV)
worin R für einen gerad- oder verzweigtkettigen Alkylrest mit 1 bis 4 Kohlenstoffatom(en) steht;
(B) - wenn X für die Carbonylgruppe steht - stereoselektive Reaktion eines Gemischs von Enantiomeren der zuvor abgebildeten allgemeinen Formel (III), worin Y und Ar die in Anspruch 1 angegebene Bedeutung besitzen und X' für die Carbonylgruppe steht, mit einem chiralen Hilfsmittel und Umsetzen des erhaltenen Produkts mit einer Verbindung der allgemeinen Formel:
R-N=C=S (IV)
worin R die in Anspruch 1 angegebene Bedeutung besitzt, sowie anschließendes Entfernen des chiralen Hilfsmittels;
(C) - wenn X für die Hydroxymethylengruppe steht und Y, R und Ar die in Anspruch 1 angegebene Bedeutung besitzen - Reduktion einer Verbindung der allgemeinen Formel (I) mit X gleich der Carbonylgruppe und Y, R und Ar gleich der in Anspruch 1 angegebenen Bedeutung;
(D) - wenn X für eine Gruppe der Formel: >C=NOR¹, >C=NN(R¹)₂ oder >C=NN(R¹)CON(R¹)₂ entsprechend der Definition von Anspruch 1 steht und Y, R und Ar die in Anspruch 1 angegebene Bedeutung besitzen - Umsetzen einer Verbindung der allgemeinen Formel (I), worin X für die Carbonylgruppe steht, mit einer Verbindung der allgemeinen Formel:
NH₂OR¹ (Va)
NH₂N(R¹)₂ (Vb) oder
NH₂N(R¹)CON(R¹)₂ (Vc)
worin R¹ die in Anspruch 1 angegebene Bedeutung besitzt, oder mit einem Säureadditionssalz hiervon;
(E) - wenn R für den Methylrest steht und X die Carbonylgruppe oder eine Gruppe der Formel >C=NOR¹ oder >C=NN(R¹)₂ entsprechend der Definition in Anspruch 1 darstellt und Y und Ar die in Anspruch 1 angegebene Bedeutung besitzen - Umsetzen von Methylamin mit einem Dithioester der allgemeinen Formel: 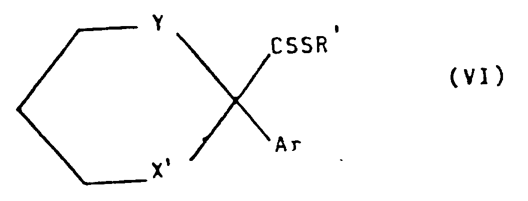 worin R' für einen gerad- oder verzweigtkettigen Alkylrest mit 1 bis 4 Kohlenstoffatom(en) oder einen Benzyl- oder Carboxymethylrest steht, X' die zuvor angegebene Bedeutung besitzt und Y und Ar die in Anspruch 1 angegebene Bedeutung besitzen;
gegebenenfalls gefolgt durch eine Stufe, in der das jeweils erhaltene Thioformamidderivat in ein pharmazeutisch akzeptables Salz desselben umgewandelt wird.

8. Arzneimittelzubereitung, umfassend ein Thioformamidderivat der allgemeinen Formel (I) gemäß Anspruch 1 oder ein pharmazeutisch akzeptables Salz desselben in Verbindung mit einem pharmazeutisch akzeptablen Träger oder Überzug.

## Patentansprüche (Patentansprüche für folgende(n) Vertragsstaat(en): ES, GR)

1. Verfahren zur Herstellung eines Thioformamidderivats der allgemeinen Formel: 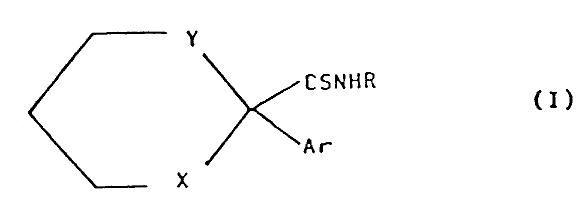 worin R für einen gerad- oder verzweigtkettigen Alkylrest mit 1 bis 4 Kohlenstoffatom(en) steht, Ar eine unsubstituierte oder in 3- und/oder 5-Stellung(en) mit einer elektronenziehenden Gruppe, ausgewählt aus einer Cyano-, Nitro-, Trifluormethyl-, Carbamoyl-, Carboxy-, C₂₋₅-Alkanoyl-, C₂₋₅-Alkoxycarbonyl- oder C₁₋₄-Alkylsulfonylgruppe oder einem Fluor-, Chlor- oder Bromatom, und gegebenenfalls weiter durch (ein) Halogenatom(e) oder (eine) C₁₋₄-Alkyl- oder C₆₋₁₂-Arylgruppe(n) substituierte Phenylgruppe bedeutet, oder die Gruppe Ar durch (ein) Halogenatom(e), (eine) C₁₋₄-Alkyl- oder C₆₋₁₂-Arylgruppe(n) oder durch zusammen einen ankondensierten Ring bildende Substituenten substituiert ist, Y einem Ethylen- oder Methylenrest oder einer Valenzbindung entspricht und X eine Carbonyl- oder Hydroxymethylengruppe oder eine Gruppe der Formeln: >C=NOR¹, >C=NN(R¹)₂ oder >C=NN(R¹)CON(R¹)₂, worin die Symbole R¹, die gleich oder verschieden sein können, jeweils für das Wasserstoffatom oder einen gerad- oder verzweigtkettigen Alkylrest mit 1 bis 4 Kohlenstoffatom(en), der unsubstituiert oder durch einen oder mehrere Substituenten, ausgewählt aus C₂₋₄-Alkenyl-, Carboxy-, C₂₋₅-Alkoxycarbonyl-, Hydroxy-, C₁₋₄-Alkoxy-, Carbamoyl-(unsubstituiert oder durch C₁₋₄-Alkylgruppen ein- oder zweifach substituiert), Amino-, C₁₋₄-Alkylamino- und Di-C₁₋₄-alkylaminogruppen, substituiert ist, oder einen Benzyl-, Phenethyl-, 1-Naphthylmethyl-, 2-Naphthylmethyl- oder Pyrid-3-ylmethylrest, von denen jeder am Ring durch ein(e) oder mehrere Halogenatom(e) oder Hydroxy-, C₁₋₄-Alkyl-, C₁₋₄-Alkoxy-(die Alkoxygruppe ist unsubstituiert oder in der für die durch R¹ darstellbaren Alkylgruppen angegebenen Weise substituiert), Cyano-, Nitro-, Trifluormethyl-, Carboxy-, C₁₋₄-Alkylamino-, C₂₋₅-Alkanoylamino- oder C₂₋₅-Alkoxycarbonylgruppen substituiert sein kann, stehen oder zwei R¹-Substituenten am selben Stickstoffatom zusammen einen gerad- oder verzweigtkettigen Alkylenrest mit 4 bis 6 Kohlenstoffatomen in der Kette, die unsubstituiert oder in der für die durch R¹ darstellbaren Alkylreste angegebenen Weise substituiert ist, bilden können, bedeutet und von pharmazeutisch akzeptablen Salzen desselben durch
(A) - wenn X für die Carbonylgruppe oder eine Gruppe der Formel >C=NOR¹ oder >C=NN(R¹)₂ mit R¹ in der obigen Definition steht, und Y, R und Ar die oben angegebene Bedeutung besitzen - Umsetzen einer Verbindung der allgemeinen Formel: 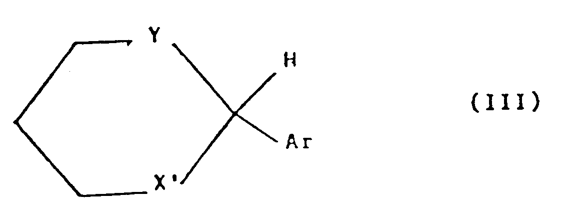 worin X' für die Carbonylgruppe oder eine Gruppe der Formel >C=NOR¹ oder >C=NN(R¹)₂ entsprechend der obigen Definition, steht und Y und Ar die oben angegebene Bedeutung besitzen, mit einem Isothiocyanat der allgemeinen Formel:
R-N=C=S (IV)
worin R für einen gerad- oder verzweigtkettigen Alkylrest mit 1 bis 4 Kohlenstoffatom(en) steht;
(B) - wenn X für die Carbonylgruppe steht - stereoselektive Reaktion eines Gemischs von Enantiomeren der zuvor abgebildeten allgemeinen Formel (III), worin Y und Ar die oben angegebene Bedeutung besitzen und X' für die Carbonylgruppe steht, mit einem chiralen Hilfsmittel und Umsetzen des erhaltenen Produkts mit einer Verbindung der allgemeinen Formel:
R-N=C=S (IV)
worin R die oben angegebene Bedeutung besitzt, sowie anschließendes Entfernen des chiralen Hilfsmittels;
(C) - wenn X für die Hydroxymethylengruppe steht und Y, R und Ar die oben angegebene Bedeutung besitzen - Reduktion einer Verbindung der allgemeinen Formel (I) mit X gleich der Carbonylgruppe und Y, R und Ar gleich der oben angegebenen Bedeutung;
(D) - wenn X für eine Gruppe der Formel: >C=NOR¹, >C=NN(R¹)₂ oder >C=NN(R¹)CON(R¹)₂ entsprechend der obigen Definition steht und Y, R und Ar die oben angegebene Bedeutung besitzen - Umsetzen einer Verbindung der allgemeinen Formel (I), worin X für die Carbonylgruppe steht, mit einer Verbindung der allgemeinen Formel:
NH₂OR¹ (Va)
NH₂N(R¹)₂ (Vb) oder
NH₂N(R¹)CON(R¹)₂ (Vc)
worin R¹ die oben angegebene Bedeutung besitzt, oder mit einem Säureadditionssalz hiervon;
(E) - wenn R für den Methylrest steht und X die Carbonylgruppe oder eine Gruppe der Formel >C=NOR¹ oder >C=NN(R¹)₂ entsprechend der obigen Definition darstellt und Y und Ar die oben angegebene Bedeutung besitzen - Umsetzen von Methylamin mit einem Dithioester der allgemeinen Formel: 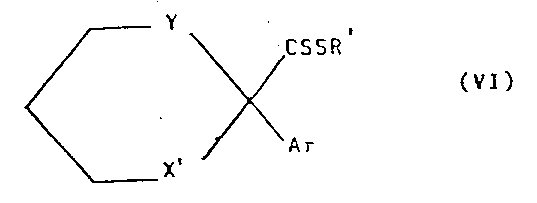 worin R' für einen gerad- oder verzweigtkettigen Alkylrest mit 1 bis 4 Kohlenstoffatom(en) oder einem Benzyl- oder Carboxymethylrest steht, X' die zuvor angegebene Bedeutung besitzt und Y und Ar die oben angegebene Bedeutung besitzen;
gegebenenfalls gefolgt durch eine Stufe, in der das jeweils erhaltene Thioformamidderivat in ein pharmazeutisch akzeptables Salz desselben umgewandelt wird.

2. Verfahren nach Anspruch 1 zur Herstellung einer Verbindung der allgemeinen Formel (I) mit X gleich der Carbonylgruppe oder einer Gruppe der Formel >C=NOR¹ in der Definition gemäß Anspruch 1.

3. Verfahren nach Anspruch 1 oder 2 zur Herstellung einer Verbindung der allgemeinen Formel (I) mit Y gleich einem Methylenrest.

4. Verfahren nach Anspruch 1, 2 oder 3 zur Herstellung einer Verbindung der allgemeinen Formel (I), worin Ar für die Phenyl-, 4-Chlorphenyl-, 3-Trifluormethylphenyl-, 3-Nitrophenyl-, 3,4-Dichlorphenyl- oder 2-Naphthylgruppe steht.

5. Verfahren nach einem der Ansprüche 1, 3 und 4 zur Herstellung einer Verbindung der allgemeinen Formel: 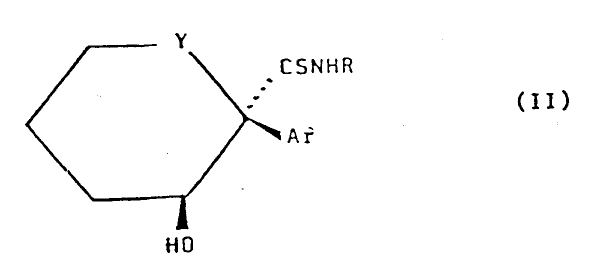 (mit Y, R und Ar in der Definition gemäß einem der vorhergehenden Ansprüche), worin sich die Hydroxygruppe in trans-Stellung bezüglich der Gruppe -CSNHR befindet.

6. Verfahren nach Anspruch 1 zur Herstellung einer Verbindung der allgemeinen Formel (I), nämlich
(±)-N-Methyl-2-oxo-1-phenylcyclohexancarbothioamid,
(±)-trans-N-Methyl-2-hydroxy-1-phenylcyclohexancarbothioamid,
(±)-anti-N-Methyl-2-hydroxyimino-1-phenylcyclohexancarbothioamid,
(±)-anti-N-Methyl-2-methoxyimino-1-phenylcyclohexancarbothioamid,
(±)-N-Methyl-2-oxo-1-(4-chlorphenyl)-cyclohexancarbothioamid,
(±)-N-Methyl-2-oxo-1-(3-trifluormethylphenyl)-cyclohexancarbothioamid,
(±)-anti-N-Methyl-2-benzyloxyimino-1-(3,4-dichlorphenyl)-cyclohexancarbothioamid,
(±)-anti-N-Methyl-2-(4-fluorbenzyloxyimino)-1-phenylcyclohexancarbothioamid,
(±)-N-Methyl-2-oxo-1-(2-naphthyl)-cyclohexancarbothioamid,
(±)-N-Methyl-2-oxo-1-(3,4-dichlorphenyl)-cyclohexancarbothioamid oder
(±)-N-Methyl-2-oxo-1-(3-nitrophenyl)-cyclohexancarbothioamid,
oder eines pharmazeutisch akzeptablen Salzes derselben.

7. Verfahren nach einem der vorhergehenden Ansprüche, gefolgt von einer Stufe der Vereinigung eines Thioformamidderivats der allgemeinen Formel (I) gemäß Anspruch 1 oder eines pharmazeutisch akzeptablen Salzes desselben mit einem pharmazeutisch akzeptablen Träger oder Überzug.

## Revendications (Revendications pour l'(les) Etat(s) contractant(s) suivant(s): AT, BE, CH, DE, FR, GB, IT, LI, LU, NL, SE)

1. Dérivé de thioformamide de formule générale : 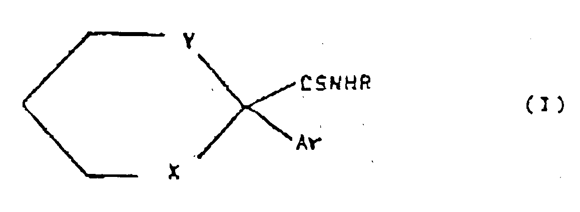 dans laquelle
R représente un radical alkyle à chaîne droite ou ramifiée en C₁-C₄, Ar représente un groupe phényle substitué ou non en position 3 et/ou 5 par un coupe attirant les électrons choisi parmi un groupe cyano, un groupe nitro, un groupe trifluorométhyle, un groupe carbamoyle, un groupe carboxy, un groupe alcanoyle en C₂-C₅, un groupe alcoxycarbonyle en C₂-C₅ ou un groupe alkylsulfonyle en C₁-C₄ ou un atome de fluor, de chlore ou de brome et facultativement substitué encore par un ou des atomes d'halogènes, un ou des groupes alkyles en C₁-C₄ ou aryles en C₆-C₁₂, ou bien un groupe Ar est substitué par un ou des atomes d'halogènes, un ou des groupes alkyles en C₁-C₄ ou aryles en C₆-C₁₂ ou par des substituants qui forment ensemble un cycle accolé, Y représente un radical éthylène ou méthylène ou une liaison de valence et X représente un groupe carbonyle ou hydroxyméthylène ou un groupe de formule >C=NOR¹, >C=NN(R¹)₂ ou >C=NN(R¹)CON(R¹)₂ dans lesquels les symboles R¹ peuvent être les mêmes ou différents et représentent chacun un atome d'hydrogène ou un radical alkyle à chaîne droite ou ramifiée en C₁-C₄ substitué ou non par un ou plusieurs substituants choisis parmi les groupes alcényles en C₂-C₄, carboxy, alcoxycarbonyles en C₂-C₅, hydroxy, alcoxy en C₁-C₄, carbamoyle (substitué ou non par un ou deux groupes alkyles en C₁-C₄), amino, alkylamino en C₁-C₄ et di(alkyl en C₁-C₄)amino, ou représentent chacun un groupe benzyle, phénéthyle, 1-naphtylméthyle, 2-naphtyméthyle ou pyrid-3-ylméthyle qui peuvent chacun être substitués au noyau par un ou plusieurs atomes d'halogènes ou groupes hydroxy, alkyles en C₁-C₄, alcoxy en C₁-C₄ (le groupe alcoxy étant substitué ou non comme pour les groupes alkyles représentés par R¹), cyano, nitro, trifluorométhyle, carboxy, alkylamino en C₁-C₄, alcanoylamino en C₂-c₅ ou (alcoxy en C₂-C₅)carbonyle ou bien deux substituants R¹ sur le même atome d'azote peuvent former ensemble un radical alkylène à chaîne droite ou ramifiée en C₄-C₆ qui est substitué ou non comme les radicaux alkyles représentés par R¹, et ses sels acceptables en pharmacie.

2. Composé selon la revendication 1, dans lequel X représente un groupe carbonyle ou un groupe de formule >C=NOR¹ tel que défini dans la revendication 1.

3. Composé selon la revendication 1 ou 2, dans lequel Y représente un radical méthylène.

4. Composé selon la revendication 1, 2 ou 3, dans lequel Ar représente le groupe phényle, 4-chlorophényle, 3-trifluorométhylphényle, 3-nitrophényle, 3,4-dichlorophényle ou 2-naphtyle.

5. Composé selon l'une quelconque des revendications 1, 3 et 4 de formule générale : 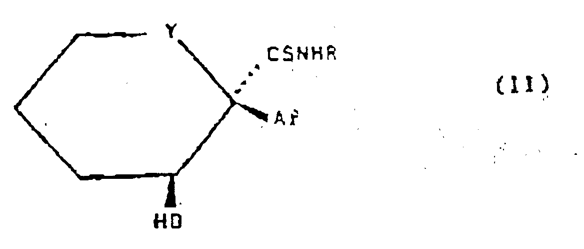 (dans laquelle Y, R et Ar sont tels que définis dans l'une quelconque des revendications précédentes) dans lequel le groupe hydroxy est en position trans par rapport au groupe -CSNHR.

6. Composé selon la revendication 1, qui est :
le (±)-N-méthyl-2-oxo-1-phénylcyclohexanecarbothioamide,
le (±)-trans-N-méthyl-2-hydroxy-1-phénylcyclohexanecarbothioamide,
le (±)-anti-N-méthyl-2-hydroxyimino-1-phénylcyclohexanecarbothioamide,
le (±)-anti-N-méthyl-2-méthoxyimino-1-phénylcyclohexanecarbothioamide,
le (±)-N-méthyl-2-oxo-1-(4-chlorophényl)cyclohexanecarbothioamide,
le (±)-N-méthy-2-oxo-1-(3-trifluorométhylphény)cylohexanecarbothioamide,
le (±)-anti-N-méthyl-2-benzyloxyimino-1-(3,4-dichlorophényl)cyclohexanecarbothioamide,
le (±)- anti-N-méthyl-2-(4-fluorobenzyloxyimino)-1-phénylcyclohexanecarbothioamide,
le (±)-N-méthyl-2-oxo-1-(2-naphtyl)cyclohexanecarbothioamide,
le (±)-N-méthyl-2-oxo-1-(3,4-dichlorophényl)cyclohexanecarbothioamide ou
le (±)-N-méthyl-2-oxo-1-(3-nitrophényl)cyclohexanecarbothioamide
ou un de ses sels acceptables en pharmacie.

7. Procédé pour la préparation d'un dérivé de thioformamide de formule générale (I) représentée à la revendication 1 qui comprend :
(A) lorsque X représente le groupe carbonyle ou un groupe de formule >C=NOR¹ ou >C=NN(R¹)₂ dans laquelle R¹ est tel que défini dans la revendication 1 et Y, R et Ar sont tels que définis dans la revendication 1, la réaction d'un composé de formule générale : 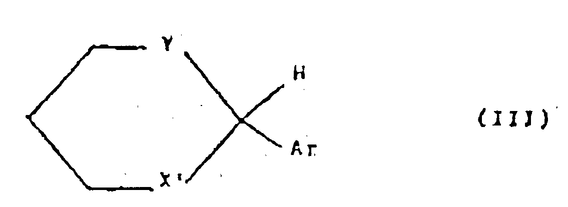 dans laquelle X' représente le groupe carbonyle ou un groupe de formule >C=NOR¹ ou >C=NN(R¹)₂ tel que défini dans la revendication 1 et Y et Ar sont tels que définis dans la revendication 1 avec un isothiocyanate de formule générale :
R-N=C=S (IV)
dans laquelle R représente un radical alkyle à chaîne droite ou ramifiée en C₁-C₄ ;
(B) lorsque X représente le groupe carbonyle, la réaction stéréosélective d'un mélange d'énantiomères de formule (III) décrite précédemment dans laquelle Y et Ar sont tels que définis à la revendication 1 et X' représente le groupe carbonyle avec un agent auxiliaire chiral et la réaction du produit obtenu avec un composé de formule générale :
R-N=C=S (IV)
dans laquelle R est tel que défini dans la revendication 1 suivie de séparation de l'agent auxiliaire chiral ;
(C) lorsque X représente le groupe hydroxyméthylène et Y, R et Ar sont tels que définis dans la revendication 1, la réduction d'un composé de formule générale (I) dans laquelle X représente le groupe carbonyle et Y, R et Ar sont tels que définis dans la revendication 1 ;
(D) lorsque X représente un groupe de formule >C=NOR¹, >C=NNY(R¹)₂ ou >C=NN(R¹)CON(R¹)₂ tel que défini dans la revendication 1 et Y, R et Ar sont tels que définis dans la revendication 1, la réaction d'un composé de formule genérale (I) dans laquelle X représente le groupe carbonyle avec un composé de formule générale :
NH₂OR¹ (Va)
NH₂N(R¹)₂ (Vb) ou
NH₂N(R¹)CON(R¹)₂ (Vc)
dans laquelle R¹ est tel que défini dans la revendication 1 ou avec un de ses sels d'addition d'acides ;
(E) lorsque R représente le radical méthyle et X représente le groupe carbonyle ou un groupe de formule >C=NOR¹ ou >C=NN(R¹)₂ tel que défini dans la revendication 1, la réaction de la méthylamine avec un dithioester de formule générale : 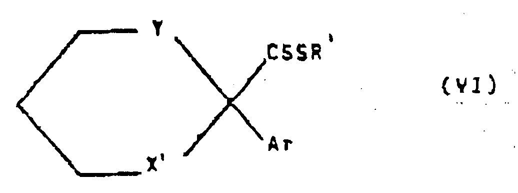 dans laquelle R' représente un radical alkyle à chaîne droite ou ramifiée en C₁-C₄ ou un radical benzyle ou carboxyméthyle, X' est tel que défini précédemment et Y et Ar sont tels que définis dans la revendication 1 ;
facultativement suivie de l'étape de conversion d'un dérivé de thioformamide ainsi obtenu en un de ses sels acceptables en pharmacie.

8. Composition pharmaceutique qui comprend un dérivé de thioformamide de formule générale (I) tel que défini à la revendication 1 ou un de ses sels acceptables en pharmacie en association avec un support du revêtement acceptable en pharmacie.

## Revendications (Revendications pour l'(les) Etat(s) contractant(s) suivant(s): ES, GR)

1. Procédé pour la préparation d'un dérivé de thioformamide de formule générale (I) : 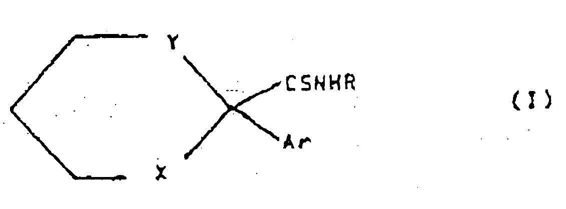 dans laquelle
R représente un radical alkyle à chaîne droite ou ramifiée en C₁-C₄, Ar représente un groupe phényle substitué ou non en position 3 et/ou 5 par un groupe attirant les électrons choisi parmi un groupe cyano, un groupe nitro, un groupe trifluorométhyle, un groupe carbamoyle, un groupe carboxy, un groupe alcanoyle en C₂-C₅, un groupe alcoxycarbonyle en C₂-C₅ ou un groupe alkylsulfonyle en C₁-C₄ ou un atome de fluor, de chlore ou de brome et facultativement substitué encore par un ou des atomes d'halogènes, un ou des groupes alkyles en C₁-C₄ ou aryles en C₆-C₁₂, ou bien un groupe Ar est substitué par un ou des atomes d'halogène, un ou des groupes alkyles en C₁-C₄ ou aryles en C₆-C₁₂ ou par des substituants qui forment ensemble un cycle accolé, Y représente un radical éthylène ou méthylène ou une liaison de valence et X représente un groupe carbonyle ou hydroxyméthylène ou un groupe de formule >C=NOR¹, >C=NN(R¹)₂ ou >C=NN(R¹)CON(R¹)₂ dans lesquels les symboles R¹ peuvent être les mêmes ou differénts et représentent chacun un atome d'hydrogène ou un radical alkyle à chaîne droite ou ramifiée en C₁-C₄ substitué ou non par un ou plusieurs substituants choisis parmi les groupes alcényles en C₂-C₄, carboxy, alcoxycarbonyles en C₂-C₅, hydroxy, alcoxy en C₁-C₄, carbamoyle (substitué ou non par un ou deux groupes alkyles en C₁-C₄), amino, alkylamino en C₁-C₄ et di(alkyl en C₁-C₄)amino, ou représentent chacun un groupe benzyle, phénéthyle, 1-naphtylméthyle, 2-naphtylméthyle ou pyrid-3-ylméthyle qui peuvent chacun être substitués au noyau par un ou plusieurs atomes d'halogènes ou groupes hydroxy, alkyles en C₁-C₄, alcoxy en C₁-C₄ (le groupe alcoxy étant substitué ou non comme pour les groupes alkyles représentés par R¹), cyano, nitro, trifluorométhyle, carboxy, alkylamino en C₁-C₄, alcanoylamino en C₂-C₅ ou (alcoxy en C₂-C₅)carbonyle ou bien deux substituants R¹ sur le même atome d'azote peuvent former ensemble un radical alkylène à chaîne droite ou ramifiée en C₄-C₆ qui est substitué ou non comme les radicaux alkyles représentés par R¹, et de ses sels acceptables en pharmacie ; procédé qui comprend :
(A) lorsque X représente le groupe carbonyle ou un groupe de formule >C=NOR¹ ou >C=NN(R¹)₂ dans laquelle R¹ est tel que défini précédemment et Y, R et Ar sont tels que définis précédemment, la réaction d'un composé de formule générale : 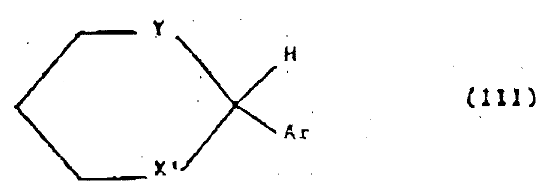 dans laquelle X' représente le groupe carbonyle ou un groupe de formule >C=NOR¹ ou >C=NN(R¹)₂ tel que défini précédemment et Y et Ar sont tels que définis dans la revendication 1 avec un isothiocyanate de formule générale :
R-N=C=S (IV)
dans laquelle R représente un radical alkyle à chaîne droite ou ramifiée en C₁-C₄;
(B) lorsque X représente le groupe carbonyle, la réaction stéréosélective d'un mélange d'énantiomères de formule (III) décrite précédemment dans laquelle Y et Ar sont tels que définis précédemment et X' représente le groupe carbonyle avec un agent auxiliaire chiral et la réaction du produit obtenu avec un composé de formule générale :
R-N=C=S (IV)
dans laquelle R est tel que défini précédemment, suivie de séparation de l'agent auxiliaire chiral ;
(C) lorsque X représente le groupe hydroxyméthylène et Y, R et Ar sont tels que définis précédemment, la réduction d'un composé de formule générale (I) dans laquelle X représente le groupe carbonyle et Y, R et Ar sont tels que définis précédemment ;
(D) lorsque X représente un groupe de formule >C=NOR¹, >C=NNY(R¹)₂ ou >C=NN(R¹)CON(R¹⁾₂ tel que défini précédemment et Y, R et Ar sont tels que définis précédemment, la réaction d'un composé de formule générale (I) dans laquelle X représente le groupe carbonyle avec un composé de formule générale :
NH₂OR¹ (Va)
NH₂N(R¹)₂ (Vb) ou
NH₂N(R¹)CON(R¹)₂ (Vc)
dans laquelle R¹ est tel que défini précédemment ou avec un de ses sels d'addition d'acides ;
(E) lorsque R représente le radical méthyle et X représente le groupe carbonyle ou un groupe de formule >C=NOR¹ ou >C=NN(R¹)₂ tel que défini précédemment, la réaction de la méthylamine avec un dithioester de formule générale : 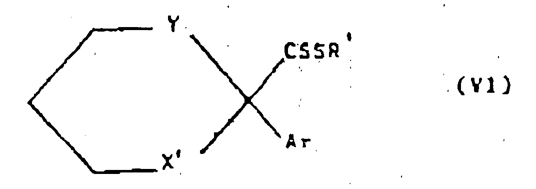 dans laquelle R' représente un radical alkyle à chaîne droite ou ramifiée en C₁-C₄ ou un radical benzyle ou carboxyméthyle, X' est tel que défini précédemment et Y et Ar sont tels que définis précédemment ;
facultativement suivie de l'étape de conversion d'un dérivé de thioformamide ainsi obtenu en un de ses sels acceptables en pharmacie.

2. Procédé selon la revendication 1, pour la préparation d'un composé de formule générale (I) dans laquelle X représente un groupe carbonyle ou un groupe de formule >C=NOR¹ tel que défini dans la revendication 1.

3. Procédé selon la revendication 1 ou 2, pour la préparation d'un composé de formule générale (I) dans laquelle Y représente un radical méthylène.

4. Procédé selon la revendication 1, 2 ou 3, pour la préparation d'un composé de formule générale (I) dans laquelle Ar représente le groupe phényle, 4-chlorophényle, 3-trifluorométhylphényle, 3-nitrophényle, 3,4-dichlorophényle ou 2-naphtyle.

5. Procédé selon l'une quelconque dès revendications 1, 3 et 4 pour la préparation d'un composé de formule générale : 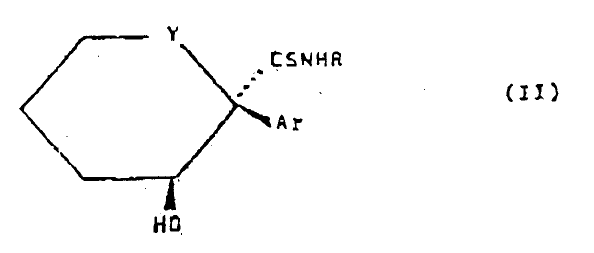 (dans laquelle Y, R et Ar sont tels que définis dans l'une quelconque des revendications précédentes) dans lequel le groupe hydroxy est en position trans par rapport au groupe -CSNHR.

6. Procédé selon la revendication 1, pour la préparation d'un composé de formule générale (I) qui est :
le (±)-N-méthyl-2-oxo-1-phénylcyclohexanecarbothioamide,
le (±)-trans-N-méthyl-2-hydroxy-1-phénylcyclohexanecarbothioamide,
le (±)-anti-N-méthyl-2-hydroxyimino-1-phénylcyclohexanecarbothioamide,
le (±)-anti-N-méthyl-2-méthoxyimino-1-phénylcyclohexanecarbothioamide,
le (±)-N-méthyl-2-oxo-1-(4-chlorophényl)cyclohexanecarbothioamide,
le (±)-N-méthyl-2-oxo-1-(3-trifluorométhylphényl)cyclohexancarbothioamide,
le (±)-anti-N-méthyl-2-benzyloxyimino-1-(3,4-dichlorophénylcyclohexanecarbothioamide,
le (±)-anti-N-méthyl-2-(4-fluorobenzyloxyimino)-1-phénylcyclohexanecarbothioamide,
le (±)-N-méthyl-2-oxo-1-(2-naphtyl)cyclohexanecarbothioamide,
le (±)-N-méthyl-2-oxo-1-(3,4-dichlorophényl)cyclohexanecarbothioamide ou
le (±)-N-méthyl-2-oxo-1-(3-nitrophényl)cyclohexanecarbothioamide
ou un de ses sels acceptables en pharmacie.

7. Procédé selon l'une quelconque des revendications précédentes, suivi de l'étape de formulation d'un dérivé de thioformamide de formule générale (I) définie dans la revendication 1 ou d'un de ses sels acceptables en pharmacie avec un support ou revêtement acceptable en pharmacie.
